(19) Europäisches Patentamt
European Patent Office
Office européen des brevets

(11) Publication number: **0 277 754**
**A1**

(12) **EUROPEAN PATENT APPLICATION**

(21) Application number: **88300652.0**

(51) Int. Cl.⁴: **A61K 49/02**

(22) Date of filing: **27.01.88**

(30) Priority: **30.01.87 GB 8702062**

(43) Date of publication of application:
**10.08.88 Bulletin 88/32**

(84) Designated Contracting States:
**AT BE CH DE ES FR GB IT LI LU NL SE**

(71) Applicant: **AMERSHAM INTERNATIONAL plc**
**Amersham Place**
**Little Chalfont Buckinghamshire HP7 9NA(GB)**

(72) Inventor: **Neirinckx, Rudi Dominique**
**c/o Dr.A.C. Peil 38 Immembachstrasse**
**CH-4125 Riehen(CH)**
Inventor: **Harrison, Roger Charles**
**East Lynne Weedon Hill**
**Hyde Heath Amersham Bucks.(GB)**

(74) Representative: **Pennant, Pyers et al**
**Stevens, Hewlett & Perkins 5 Quality Court**
**Chancery Lane**
**London, WC2A 1HZ(GB)**

(54) **Method involving propylene amine oxime complexes.**

(57) Neutral lipophilic complexes of propylene amine oxime ligands have the property of undergoing in vivo a chemical reaction mediated by glutathione resulting in loss of the ability to pass through cell membranes. This property is used to detect or treat regions of relatively high in vivo glutathione concentration. Complexes of propylene amine oxime ligands having the formula 3 in the d,l-or particularly the meso-form, may be used to detect or treat tumours or other damaged or diseased tissue.

EP 0 277 754 A1

Xerox Copy Centre

# METAL COMPLEXES FOR DIAGNOSIS AND THERAPY

European Patent Specification 123504 provides a lipophilic macrocyclic complex of Technetium-99m useful as a diagnostic radiopharmaceutical which can be formed by complexing in aqueous solution Tc-99m pertechnetate under reducing conditions with an alkylene amine oxime containing 2 or 3 carbon atoms in the alkylene group, which group is unsubstituted or substituted, the complex having a core with a zero net charge, containing an O-H-O ring closure bond, and being sufficiently stable for parenteral administration and imaging by scintilation scanning, any alkylene substituents present being of the kind useful for adopting radionuclide ligands for body imaging applications. Preferred complexes are believed to have the formula 1:-

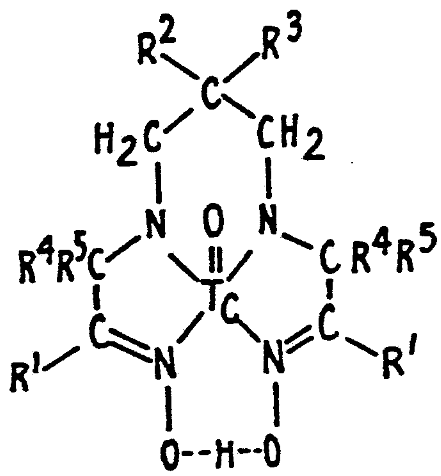

1

where each $R^1$, $R^4$, and $R^5$ is hydrogen or C1 to C12 alkyl, and each of $R^2$ and $R^3$ is hydrogen, hydroxyl, C1 to C12 alkoxyl, C1 to C22 hydrocarbon which may be alkyl, alkenyl, alkaryl, aralkyl or aryl, or tertiary amine with 1 to 20 carbon atoms, or $R^2$ and $R^3$ form, together with the carbon atom to which they are attached, a cycloaliphatic group which may be amine substituted.

EPA 194843 relates to a family of complexes, and the associated ligands, which complexes show interesting properties particularly as regards brain retention. That specification describes propylene amine oxime ligands, which are capable of forming lipophilic macrocyclic complexes with technetium-99m, and having the formula 2:-

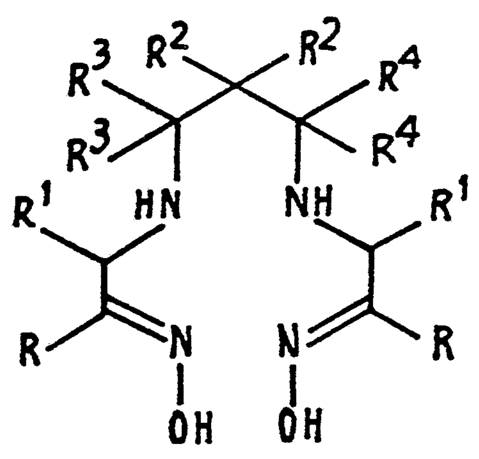

2

where R is H, alkyl, aryl, cycloalkyl, $CF_3$, $CONX_2$ or $C_6H_4OCH_3$.

R is alkyl, aryl, cycloalkyl or $CF_3$.

or R and $R^1$ together form part of a cycloalkyl ring,

each of $R^2$, $R^3$ and $R^4$ is H, alkyl, aryl, cycloalkyl or $CF_3$, and

X is H, $CH_3$, $C_2H_5$ or $C_6H_5$,

wherein the ligand of formula 2 is in the form of a single stereoisomer or of a mixture of 2 or more such stereoisomers.

An important member of this family is the ligand having the formula 3:-

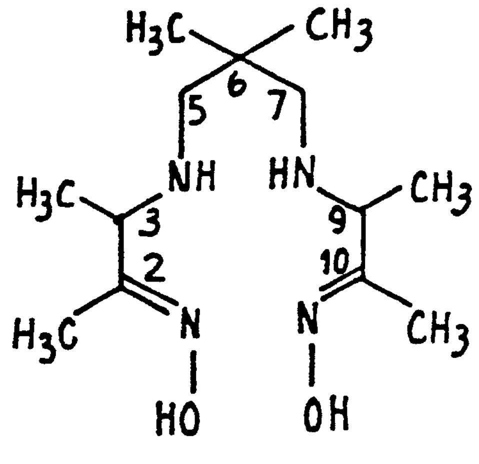

3

This ligand, together with some other ligands according to formula 2, have two asymmetric carbon atoms and thus exist in the form of three stereoisomers. The meso-diastereoisomer has distinctly different properties from the d,l-diastereoisomer (a racemic mixture of the d-and l-enantiomers). Furthermore, the Tc-99m complexes of the two diastereoisomers have markedly different in vivo properties. Furthermore, the d- and l-enantiomers of the ligands have properties (physical and biological) which differ from each other, and from a racemic mixture of the two enantiomers and from the meso-diastereoisomer. The different in vivo properties of the Tc-99m complexes of these isomers is surprising.

EPA 179608 relates to another family of complexes, and the associated ligands, which complexes also show interesting properties as regards brain retention. The ligands have the general formula:-

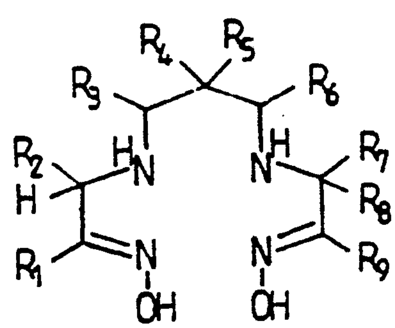

wherein each of $R_1$, $R_2$, $R_3$, $R_4$, $R_5$, $R_6$, $R_7$, $R_8$ and $R_9$ is independently hydrogen, $C_1$-$C_8$ straight-or branched-chain saturated or unsaturated alkyl, aralkyl, aryl, alkoxy, alkoxycarbonyl, alkylcarbonyl, alkylcarbonyloxy, primary secondary or tertiary amine or amide, or nitrile,
or one or more of $R_1$, $R_2$; $R_3$, $R_4$; $R_5$, $R_6$; $R_7$, $R_8$; $R_8$, $R_9$ are joined to form a fused or spiro carbocyclic or heterocyclic ring,
provided that, when $R_1$ is the same as $R_9$ and $R_2$ is the same as $R_7$, then $R_8$ is not hydrogen. These compounds are characterized by being asymmetric about the central carbon atom (to which $R_4$ and $R_5$ are attached). If $R_2$ is not hydrogen, they also exist in the form of two (or more) stereoisomers.

The ability of molecules with a zero charge to diffuse through biological membranes is related in a positive way to lipophilicity. But such diffusion is expected to take place in both directions at about the same rate. It has been proposed that the ability of propylene amine oxime complexes to become trapped in particular organs in the body may arise as a result of reaction of the complexes in those organs in such a way as, either to destroy the zero charge of the complex by creating an ionic species, or to reduce the lipophilicity of the complex. Hitherto, no satisfactory explanation for this phenomenon has been put forward.

This invention results partly from our discovery that the biodistribution of Tc-99m complexes of propylene amine oxime ligands is consistent with reaction of these complexes in vivo, with or mediated by glutathione, to form non-diffusable species. Glutathione, a tripeptide, gamma-H-glutamyl-L-cysteinylglycine,

is a ubiquitous component of animal tissues. Since free cysteine is present in only trivial quantities, glutathione is the most abundant sulfhydryl compound in cells and appears to function in maintaining many enzymes in their active conformation. The compound is present in nM concentrations and constitutes the bulk of all free thiols in the typical mammalian cell. See Kosower, N.S., The Glutathione Status of Cells, Int. Rev. Cytol, 54, 109-160, 1978, who states on page 115 that GSH is mostly free and states ranges for intracellular GSH (0.5-10 mM) and GSSG (5-50uM).

Our hypothesis is based on the instability of all Tc-99m propylene amine oxime complexes (formula 1), which is more pronounced in the demethylated series of formula 2 of which the compound of formula 3 is an example. The complexes have very different stabilities even in aqueous media containing only the complex, excess ligand and a small amount of stannous chloride. Compare, for example, PnAO (formula 1 where $R^1$, $R^4$, and $R^5$ are methyl and $R^2$ and $R^3$ are H); meso HM-PAO (the meso-isomer of compound 3); and d,l HM-PAO (the d,l isomer of compound 3). Under comparable conditions, the relative rates of disappearance of the lipophilic complexes of PnAO, meso HM-PAO and d,l-HM-PAO are approximately 1, 4 and 30 respectively. Although the in vitro reactions are not fast enough to account for the in vivo retention of Tc-99m HM-PAO complexes the degree of trapping in the brain generally follows the order of this instability. PnAO is retained only to a small extent, meso-HM-PAO has an average of about 1.7% retention and dl-HM-PAO has about 5% retention in human brain. The conversion of the lipophilic species in aqueous media is greatly accelerated by the addition of glutathione.

Based on the rate of reaction between Tc-99m complexes of propylene amine oxime ligands and glutathione, and on the known glutathione concentrations of various organs, we have calculated the percent retention of complexes in these organs on the basis of our theory. The following are examples of the results obtained with d,l-HM-PAO.

| Organ | Percent Retention Calculated | Percent Retention Clinical |
|---|---|---|
| Brain | 4.1 | 4.9± 1.3 |
| Heart | 0.9 | 1.5± 0.5 |
| Liver | 14 | 12 ± 4.4 |

The agreement between the theoretical quantitative organ retention, based on the assumption that the trapping rate is controlled by a glutathione reaction with the complexes, and the clinical data, provides satisfactory support for the proposed mechanism. This does not exclude minor contributions to the conversion from other intracellular components, but the comparatively high glutathione concentrations and known reaction rate account for most of the trapped radioactivity.

By virtue of being neutral and lipophilic, the complex has the ability in vivo to pass through cell membranes, which ability is lost on reaction with, or mediated by, glutathione. The reaction is readily monitored in vitro by providing an aqueous solution of the gluathione and the ligand containing also a reducing agent such as a stannous salt, and adding pertechnetate ($TcO_4$ ) eluate from a technetium generator. The reaction goes readily at ambient or elevated temperature; at 37°C, a reaction rate of 208 l mol ¹ min ¹ was measured.

In one aspect, the invention provides a method of preparing a formulation for the detection or treatment of regions of relatively high (or low) glutathione concentration, which method comprises bringing into solution a neutral lipophilic complex of a propylene amine oxime ligand having the property of undergoing in vivo a chemical reaction mediated by glutathione resulting in loss of the ability to pass through cell membranes. The ligand may be as described in EPA 123504, but is more preferably according to formula 2 above, for example an isomer of formula 3. The complex is generally with Tc-99m, but may be with other radioactive or non-radioactive transition metals as described below.

The term "a chemical reaction mediated by glutathione" is used because we are not certain precisely what the chemical reaction involved may be. Glutathione exists in vivo mainly in a reduced or sulphydryl form, but also to a small extent in an oxidized form which shows no significant reactivity with the PnAO

complexes. Most of the reduced form is attached though not covalently bonded to protein, but a small proportion exists in the free state. Reaction of the PnAO transition metal complex with, or mediated by glutathione, appears to involve, or to be rapidly followed by, attachment of the transition metal to a marcromolecule which may in some cases be proteinaceous with a molecular weight of around 60000 and which effectively immobilises the transition metal. Probably the reaction takes place, at least to a major extent, between the complex and glutathione. But there may also be chemical reaction between the complex and other sulphur-containing amino acids and polypeptides present in relatively low concentration.

The determination of glutathione concentration has not previously been regarded as a matter of very great clinical interest. But there are some abnormal or diseased states which can give rise to abnormal glutathione concentrations in particular locations. So a measurement of glutathione concentration can provide indirect evidence of an abnormal or diseased state. This is more fully discussed below.

In another aspect, the invention provides a method involving the use of a neutral lipophilic complex of a propylene amine oxime ligand having the property of undergoing in vivo a chemical reaction mediated by glutathione resulting in loss of the ability to pass through cell membranes, which method comprises administering to a patient the neutral lipophilic complex which undergoes the chemical reaction mediated by glutathione and as a result becomes located in vivo in regions of relatively high (or low) glutathione concentration.

The ligand may be as described in EPA 123504, more preferably a ligand of formula 2 above, such as an isomer of formula 3. For in vivo diagnostic purposes, the metal of the complex is generally Tc-99m. For therapeutic purposes, there may be used some other radioactive metal such as the beta-emitting rhenium-186. The complex is preferably administered by intravenous injection. Conditions for administering d,l-HM-PAO Tc-99m complex are described in the literature, and conditions for administering other PnAO complexes are readily determined by routine testing.

Retention of the complex by a region is related to blood flow past the region and also to glutathione concentration in the region. Specifically, the ratio between the conversion rate (of the complex into an immobile species) and the blood flow rate determines whether organ retention is mainly determined by blood flow or by glutathione concentration. These effects are difficult to separate, so that increased retention may sometimes be wrongly ascribed to increased blood flow.

It appears that tumours in general may have abnormally high glutathione concentrations. The invention may therefore provide a method for the screening and detection, or alternatively for the treatment, of tumours or other damage or disease. Particularly for screening and detection, the method should be much quicker, simpler and cheaper than other methods.

In yet another aspect, the invention provides a method of detecting or treating tumours or other damaged or diseased tissue, which method comprises administering to a patient a neutral lipophilic complex of a propylene amine oxine ligand of formula 3 in its meso form. Various specific instances are described below.

Smokers have a high lung-retention of d,l HM-PAO, and the degree of retention may be of prognostic value. Screening of heavy smokers becomes a possibility.

A) Diagnosis and staging of lung tumours.

Human lung carcinoma cell line A.549 has a very high glutathione content [Brodie A E and Reed D J, Toxicol.Appl.Pharmacol. 77(3), 381-7, 1985]. The concentration given was 25+5nmol/$10^6$ cells. The cell volume is $5x10^{-13}$ l. This leads to a glutathione concentration of 50±10mM. Normal lung tissue is 1.5mM in GSH. With a flow of 5-10min$^{-1}$, the calculated trapping fractions for d,l HM-PAO are 0.05-0.1 in normal tissue and 0.6-0.8 in tumour. For meso-HM-PAO the calculated trapping fractions are 0.005-0.01 for normal lung and 0.14-0.24 for tumour. Thus, d,l HM-PAO is expected to have a very high retention in lung tumours but meso-HM-PAO may be expected to show better contrast with normal tissue.

Brodie and Reed observe that cell line A.549 has a higher GSH content than other neoplastic cells. They report that HeLa cells (a cervical cancer strain) have a glutathione content of 4.8nmol/$10^6$ cells (=10mM). This is still notably higher than for normal tissue. A.549 is regarded as an excellent model for lung carcinoma. Thus, there are grounds for believing that lung tumours in general have a very high glutathione content.

B) Liver cirrhosis and glutathione concentration are related. Glutathione scanning may be an early indicator of tissue damage, and may be able to detect it before it reaches the incurable stage.

Shi E.C.P. et al (Factors Influencing Hepatic Glutathione Concentrations: A study in Surgical Patients, Clin. Sci., 62, 279-283, 1982) find control values ([GSH])=3.9mM which are close to 0.5-10 the normal

range for animals (0.5-10mM). They found that any of the four risk factors, abnormal histology, abnormal postoperative liver function, drug ingestion and protein malnutrition, reduced hepatic GSH. There is a variety of papers on the effect of ethanol treatment on animal hepatic GSH. Typical is Fernandex V. and Videla L.A., Effect of Acute and Chronic Ethanol Ingestion on the Content of Reduced Glutathione of Various Tissues of the Rat, Experientia, 37(4), 392-394, 1981. They find hepatic GSH to be reduced by both acute and chronic ethanol treatment.

C) Brain anomalies.

The long residence time of Tc-99m d,l HM-PAO in brain makes it suitable for use with a rotating gamma camera, the most widely-used SPECT instrument. As such the compound promises to become the preferred choice for the study of brain blood flow abnormalities in man and clinical evaluation in many European countries has confirmed its capability: Alzheimer's disease, multi-infarct dementia, stroke, transient ischemic attack, trauma, epilepsy and migraine have all been demonstrated by SPECT using Tc-99m d,l HM-PAO.

Tumours and neurological pathology may be associated with glutathione concentration. Since glutathione is linked with NADH, the knowledge that glutathione forms adducts with Tc-99m d,l HM-PAO may give additional information about brain metabolism.

D) Glutathione concentration may be indicative of tumour formation in general. As regards GSH-levels in a variety of tumours, Int.J.Rad.Oncol., 12(7), 1986, gives the following information on page 1144:

| Cell Type | Strain | [GSH] nmol/mg Protein |
|---|---|---|
| Human lung'adenocarcinoma | A549 | 324 |
| Human large cell carcinoma | NCl-H460 | 149 |
| Human Cervical carcinoma | HeLa | 103 |
| Human melanoma | Ul | 88 |
| Normal human skin fibroblast | 1522 | 32 |
| Chinese hamster carcinoma | V79 | 25 |

E) Anti-tumour activity.

If the metal is retained in glutathione-rich tumour tissue, it may be possible to use the radioactive or non-radioactive metal of the complex, not for diagnosis and detection, but for in situ treatment of the tumour. For this purpose, beta-emitting isotopes such as rhenium-186 are candidates.

EXAMPLE 1

This example concerns tumour uptake studies and is a comparison of $Tc^{99m}$-meso-HMPAO (the meso isomer of the ligand of formula 3) with $Tc^{99}$-d,l-HMPAO (the d,l-isomer of the ligand of formula 3), and the concentration of GSH, denoted [GSH]. Tc-99m-meso-HMPAO and Tc-99-d,l-HMPAO were co-injected in rats and dissection performed one hour post injection. Samples were counted immediately for Tc-99m and then heart and tumour samples were homogenised for [GSH] determination. 1ml aliquots of homogenate were digested for Tc-99 counting. Tc-99 was counted when Tc-99m had decayed (4 to 5 days due to the high counting efficiency of the liquid scintillant for Tc-99m).

Three rat tumours were studied, and the data is summarized in the accompanying Table. The data indicates that Tc-99m-meso-HMPAO does show significantly higher tumour uptake than Tc-99-d,l-HMPAO.

Comparisons of $Tc^{99m}$-Meso-HMPAO with $Tc^{99}$-d,l-HMPAO and [GSH] in Rats bearing Tumours

| Tumour | $Tc^{99m}/Tc^{99}$ Ratio in A Tumour | $Tc^{99m}/Tc^{99}$ Ratio in B Heart | Tumour/ Heart (A/B) | [GSH] Tumour/Heart |
|---|---|---|---|---|
| HSN rat fibrosarcoma | 2.6 | 0.45 | 5.8 | 1.1 |
| MC 24 rat fibrosarcoma | 2.1 | 0.38 | 5.5 | 0.86 |
| RAH3 rat hepatoma | 3.2 | 0.32 | 10.0 | 1.4 |

(All results are the average of six rats)

Example 2

The following experimental section describes a theoretical model for the in vivo behaviour of the Tc-99m d,l-HM-PAO, and provides a demonstration that reaction with intracellular free thiols, such as glutathione, contributes substantially to the conversion of Tc-99m HM-PAO complexes in human tissue and is the rate determining step for the trapping process.

MATERIALS

The Tc-99m d,l HM-PAO complex was prepared using a freeze-dried formulation (Ceretec™*) following the manufacturer's instructions. Tc-99m meso HM-PAO was prepared from a similar formulation in which the meso form of HM-PAO replaced the d,l diasteroisomer. Glutathione was obtained from Sigma.

* Amersham International plc.

METHODS

1) Calculation of the expected in-vivo distribution

a) In-vivo model

Our model for the in-vivo behaviour of Tc-99m d,l HM-PAO is shown in Fig. 1.

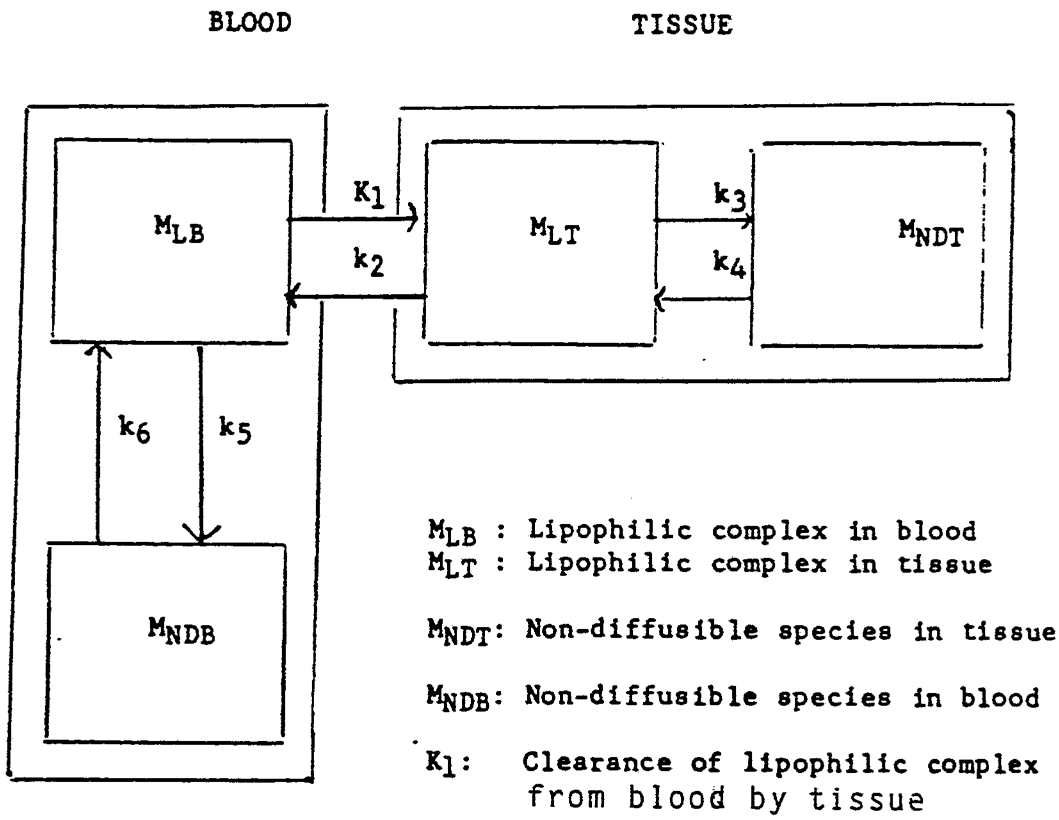


$M_{LB}$ : Lipophilic complex in blood
$M_{LT}$ : Lipophilic complex in tissue

$M_{NDT}$: Non-diffusible species in tissue

$M_{NDB}$: Non-diffusible species in blood

$K_1$: Clearance of lipophilic complex from blood by tissue

$k_2$-$k_6$: First order rate constants, describing the transport of the Tc-99m between compartments

We consider the first hour after injection of the complex. The converted material is assumed to be trapped giving $k_4 = k_6 = 0$. The division of blood into just two compartments, representing freely diffusible and trapped material, represents a simplification of a very complex system. Red cells contain the blood glutathione pool(16). Experimental evidence suggests that red cells are the principal site of trapping in blood and plasma protein binding is low(17).

The clearance of lipophilic material from blood by tissue is given by

$K_1 = fE$

where f is the tissue perfusion (volume flow rate per unit mass of tissue) and E is the extraction efficiency. E is given by Crone(18) as

$E = l - e^{-PS/f}$

where S is the capillary specific surface area and P the permeability coefficient for lipophilic complex.

The first order rate constant $k_2$ describes the wash-out of lipophilic complex from tissue to blood. We assume the lipophilic complex to be freely diffusable so

$k_2 = \rho K_1 = \rho fE$

where $\rho$ is the density of the tissue.

The pseudo-first order rate constants $k_3$ and $k_5$ describe the convention of lipophilic complex in organ tissue and blood respectively. If the conversion is caused by reaction with glutathione, then

$k_3 = k_{GSH}\,[GSH]_{tissue}$

$k_5 = k_{GSH}\,[GSH]_{tissue}$

where $k_{GSH}$ is the second order rate constant describing the reaction of lipophilic complex with GSH, $[GSH]\uparrow_{tissue}$ is the concentration of GSH in tissue and $[GSH]\uparrow_{blood}$ is the average concentration of GSH in whole blood. The value of $k_5$ is independent of the distribution of GSH amongst blood components. Since GSH is present in vast excess compared to the no carrier-added Tc-99m complex, we assume its concentration to

be undisturbed by the reaction.

b) Calculation of the retained fraction in each organ

The fraction (R) of the lipophilic molecules entering the organ and retained there can be calculated as follows:-

The fraction retained

$$R = \lim_{t \to \infty} \frac{N_{trapped}}{N_o}$$

$N_o$ = The total number of lipophilic molecules originally present in the organ
= $N_{free}$ + $N_{trapped}$ + $N_{washed\text{-}out}$
and $N_{free}$, $N_{trapped}$ and $N_{washed\text{-}out}$ respectively represent the numbers of molecules freely diffusible, trapped and washed-out at time t

$$\frac{dN_{free}}{dt} = -(k_3 + k_2).N_{free}$$

$N_{free} = N_o. e^{-(k_3 + k_2)t}$

$$\frac{dN_{trapped}}{dt} = k_3.N_{free}$$

$$N_{trapped} = k_3N_o \int_o^{\infty} e^{-(k_3 + k_2)t} dt$$

$$= \frac{k_3N_o}{k_3 + k_2}$$

$$R = \frac{N_{trapped}}{N_o} = \frac{k_3}{k_3 + k_2}$$

c) Proportions retained in each organ

The first pass in-vivo distribution can be calculated as
$D = L.e^{-k_5T}.C.V.E.R$
where:
D is the fraction of the injected dose retained in the organ;
L is the radiochemical purity of the Tc-99m HM-PAO preparation at the time of injection;
$e^{-k_5T}$ is the fraction of injected lipophilic complex remaining unconverted in blood at time T, the delay time from injection site to organ;
C is the proportion of the total cardiac output delivered to the organ;
and V is the fraction of injected lipophilic complex remaining after passage through other vascular tissue before reaching the organ under study. We seek to calculate the organ distribution following injection by the intravenous route. Consequently, for lungs V = 1 and for brain and heart V = 1-E.$R_L$, where $R_L$ is the

proportion retained in lung. The liver receives about 75% of its blood supply through the portal vein(19). This supply will have lost a fraction $E.R_G$ of lipophilic complex to the gastrointestinal tract.
Consequently, for liver,
$V = (1-E.R_L)(1-0.75.E.R_G)$

### 2) In-vitro measurement of reaction constants, $^{k}GSH$

5mM glutathione in 0.01M pH 7.4 phosphate buffer was prepared under a nitrogen atmosphere and diluted with $N_2$ purged buffer to give samples varying from 0.01 mM to 0.5 mM in GSH. 5 ml samples were transferred into a total of 17 $N_2$-filled vials. In addition 5 vials of phosphate buffer were used as controls. These were incubated at 37°C in a water bath. Vials of Ceretec™ were reconstituted with pertechnetate according to the manufacturer's instructions (1.1 GBq Tc-99m per 5 ml) and 0.5 ml aliquots of the Tc-99m complex solutions were injected into the glutathione or buffer-containing vials. Samples were taken from the vials at 2', 15', 30', 45' and 60' after mixing and subjected to the standard Ceretec™ RCP measurement by TLC and to HPLC (PRP column in phosphate/THF mixture)(5). Samples of the inactive glutathione solutions were assayed spectrophotometrically during the course of the experiment according to the method of Grassetti and Murray(20).

For each vial the radiochemical purity measurements at each time point were used to calculate the first order rate constant, k, for the disappearance of lipophilic complex. A plot of k against [GSH] gave $k_{GSH}$ from $k = k_{GSH}[GSH]$. The range of GSH concentrations employed was chosen because the disappearance of lipophilic complex was found to be too fast for convenient measurement at values of [GSH] comparable to those found intracellularly.

The value of $k_{GSH}$ for the meso isomer was obtained from a comparative study of the rate of reaction of d,l and meso isomers.

## RESULTS

### 1) Determination of $^{k}GSH$

The results of the spectrophotometric assays showed that the free thiol concentrations remained unchanged during the course of the experiment. The radiochemical purity measurements demonstrated that, in the presence of glutathione, one of the reaction products of lipophilic complex was material which interfered with the determination of the proportion of lipophilic complex. Accordingly, for each vial, upper and lower limits for k(d,l) were determined in order to allow for the uncertainty of the radiochemical purity determinations. Both the upper and lower values of k were included in the plot of k against [GSH] and a least mean squares analysis gave $k_{GSH} = 208 \pm 20$ (standard error) l $mol^{-1}$ $min^{-1}$. Straight lines were also drawn through the extreme points of the plot and upper and lower limit values for $k_{GSH}$ of 341 and 122 l $mol^{-1}$ $min^{-1}$ respectively determined. For the meso isomer the rate was found to be 11.4 times slower than for d, l. This gave a mean value for $k_{GSH}$(meso) of 18.2 l $mol^{-1}$ $min^{-1}$ with upper and lower limits of 29.9 and 10.7 l $mol^{-1}$ $min^{-1}$ respectively.

### 2) Derivation of parameters used for calculating organ distributions

The values of f, E, $\rho$, $k_2(=\rho fE)$, T and [GSH] used for calculating the organ distributions are listed in Table 1.

Values for f were obtained from Davson and Eggleton(21). Lassen et al(22) have determined PS for d, l HM-PAO entering human brain as 1.25 $mlg^{-1}$ $min^{-1}$ Hence, E was calculated from Crone's equation as 0.90. The brain is known to have both a lower permeability(23) and lower capillary surface area(24) than other organs. Consequently, the extraction efficiencies for d,l lipophilic complex entering heart, liver and gastrointestinal tract have been taken as 1.0. The high flow in lung renders the assumption of E = 1.0 for lungs as dubious. However, for the calculations presented below, a change from E = 1.0 ($PS/f \rightarrow \infty$) to E = 0.2 (PS = 2.5 $mlg^{-1}$ $min^{-1}$, ie twice that of brain) produces, at worst, only a 15% decrease in calculated lung radioactivity and a minute change in $(1-E.R_L)$. Consequently, the approximation E = 1.0 for lungs is employed. We note that the true value of E may differ substantially from 1.0. As the membrane permeability of a passively transported molecule depends mainly upon its size and lipophilicity the E values for the

meso lipophilic complex have been taken as equal to those used for the d,l lipophilic complex. The calculated organ radioactivities for the meso lipophilic complex are even less sensitive to the value used for E for lungs than are those of the d,l lipophilic complex. The tissue densities ($\rho$) were derived from the ICRP Report on Reference Man(25) except for the density of liver which was estimated as 1.04 g $ml^{-1}$. Delay times (T) were derived from the clinically measured transit time from injection site to each organ. Literature values for human organ glutathione concentrations are obtained either from biopsy (ie diseased tissue) or post mortem samples. These were considered to be unrepresentative of live, normal volunteers. However, reported values for rat, mouse, guinea pig and rabbit(26-35) show little interspecies difference and so the human values have been estimated by averaging those reported for these animals.

The results described above were used in calculating values for the rate constants $k_3$ and $k_5$ and retained fractions, R. These are listed in Table 2 for the full range of determined $K_{GSH}$ values. The value for [GSH] in whole blood was taken as 1.2 mM(16).

3) Calculation of the expected human biodistribution and comparison with clinical results

Table 3 describes the calculation of the human organ distributions using the formula

$D = L.e^{-k_5.T}C.V.E.R$

derived above and compares the calculated values with clinical results(4,14).

The fractional cardiac outputs (C) to heart, lung and liver were derived from Winton and Bayliss (19). The small contribution of the bronchial circulation to C for lung has been ignored. There is much disagreement in the literature on the magnitude of the fractional cardiac output to brain. We have employed a value of 12% derived from values of brain weight and total cardiac output(25) and f. The values used for L, the radiochemical purity, are derived from experience of the behaviour of Tc-99m HM-PAO complexes-(12) in the conditions employed in the clinical study(4,14).

The clinical studies were all conducted in normal volunteers. The reported percentages retained in each organ are all corrected for intravascular radioactivity. The clinical data for Tc-99m d,l HM-PAO covered four European centres and a total of 22 normal volunteers(14). The clinical data for Tc-99m meso HM-PAO were derived from two normal volunteer studies at the Aberdeen Royal Infirmary(4). The radioactivity in heart was estimated by inspection of whole body images at the participating centres.

For Tc-99m d,l HM-PAO it is seen that there is good agreement between calculated and found organ distributions. For lung the agreement appears less good than that found for other organs. However, it should be noted that most of the measured lung activities were not corrected for soft tissue background and some of the normal volunteers were smokers and, as reported by Sharp(4), more likely to show high lung radioactivities. When these qualifications are considered it is seen that calculated and measured lung radioactivities may be reconciled. Overall, the agreement between calculated and observed organ distributions is best for the middle to higher end of the range of possible $k_{GSH}$values.

For Tc-99m meso HM-PAO, the size of the clinical sample (two volunteers) is too small to justify a detailed comparison of calculated and measured distributions. However, it is seen that the proposed trapping mechanism accounts for a substantial proportion of the measured brain radioactivities.

All calculated organ uptakes depend strongle on the values chosen for C and L and hardly at all on T. For all organs with the meso isomer and for lungs with the d, l isomer

$k_2 \gg k_3$

$R \sim k_3/k_2$

In these cases R is small and strongly dependent on $k_{GSH}$, [GSH] and f. As the radioactivity entering the organ is proportional to C, the retained radioactivity depends on $k_{GSH}$ and [GSH]. For the other organs with the d, l isomer R is weakly dependent on $k_{GSH}$, [GSH] and f and the calculated organ retentions are relatively insensitive to variations in the values chosen for these variables.

## REFERENCES

(1) Neirinckx R D, Nowotnik D P, Pickett R D, Harrison R C, Ell P J.
Development of a Lipophilic TC-99m Complex Useful for Brain Perfusion Evaluation with Conventional SPECT Imaging Equipment. In: Amphetamines and pH-shift agents for brain imaging: Basic Research and clinical results. Proceedings of a workshop of the Rheinisch West-Fachische Gesellschaft fur Nuklearmedizin. Eds: H Biersack and C Winkler, Walter de Gruyter Publishers, Bonn, 1984. P59-70.

(2) Ell P J, Jarritt P H, Cullum I, Hocknell J M L, Costa D C, Lui D, Jewkes R F, Steiner T, Nowotnik D P, Pickett R D, Neirinckx R D.
A new Regional Cerebral Blood Flow Mapping with a Tc-99m labelled Compound. The Lancet, 6.7.85, P50.

(3) Nowotnik D P, Canning L R, Cumming S A, Harrison R C, Higley B, Nechvatal G, Pickett R D, Piper I M, Bayne V J, Forster A M, Weisner P A, Neirinckx R D.
Development of a Tc-99m-Labelled Radiopharmaceutical for Cerebral Blood Flow Mapping. Nucl. Med. Commun. 1985, 6, 499-506.

(4) Sharp P F, Smith F W, Gemmell H G, Lyall D, Evans N T S, Gvozdanovic D, Davidson J, Tyrrell D A, Pickett R D, Neirinckx, R D
99mTc HM-PAO Stereoisomers for Imaging Regional Cerebral Blood Flow - Human Volunteer Studies.
J. Nucl. Med. 1986, 27,171-177.

(5) Neirinckx R D, Canning L R, Piper I M, et al.
Tc-99m d, 1 HM-PAO: A New Radiopharmaceutical for SPECT Imaging of Regional Cerebral Blood Perfusion.
J. Nucl. Med. (in the press).

(6) Winchell H S, Baldwin R M, Lin J H.
Development of I-123 Labelled Amines for Brain Studies.
Localisation of I-123 Iodophenylalkylamines in Rat Brain.
J. Nucl. Med. 21, 940-946, 1980.

(7) Tramposch K M, Kung H F, Blau M. Radioiodine labelled N,N-Dimethyl-N′ -(2-hydroxy-3-alkyl-5-iodobenzyl)-1,3-propanediamine s for Brain Perfusion Imaging. J. Med. Chem. 26, 121-125, 1983.

(8) Wyth A, Fennema P J, Van de Shoot J B. T1-201 Diethyldithiocarbamate: A possible Radiopharmaceutical for Brain Imaging. Pharm. weekblad scientific. Edn. 5, 213-216, 1983.

(9) Biersack H J, Reichmann K, Winkler C, Stefan H, Kuhenen K, Bulau P, Penin H, Tyrrell D A, Neirinckx R D, Gruner K R.
Brain SPECT with $^{99m}$Tc-Labelled Hexamethyl Propylene Amine Oxime (HM-PAO) in patients with Epilepsy. Lancet 21.12.85.76.

(10) Ell, P J, Hocknell, J M L, Jarritt, P H, Cullum, I, Lui, D, Nowotnik, D P, Pickett, R D, Canning, L R, Neirinckx, R D.
A 99m Tc-Labelled Radiotracer for the Investigation of Cerebral Vascular Disease. Nucl. Med. Commun. 1985, 6, 437-41.

(11) Biersack H, Reichmann K, Stephan H, Kuhnen U, Neirinckx R D, Tyrrel D A, Penin H, Winkler C. Brain SPECT with $^{99m}$TC-labelled Hexamethyl Propylene Amine Oxime (HM-PAO): Results in Epilepsy. Radioaktive Isotope in Klinik und Forschung, 17 Band (1986), 33-38.

(12) Forster, A M. Unpublished results.

(13) Holm S, Andersen A R, Vorstrup S, et al. Dynamic SPECT of the Brain using a Lipophilic Technetium-99m Complex, PnAO.
J. Nucl. Med., 26, 1129-1134, 1985.

(14) Tyrrell D A. 99m Tc HM-PAO - European Phase I Clinical Trials, Amersham International Report N109/28, 4 December 1985.

(15) Kosomer E M. Chemical Properties of Glutathione in Glutathione: Metabolism and Function. Arias J M and Jakoby W B (Eds.), Raven Press, New York, 1976, pl.

(16) Tietze F. Enzymic Method for Quantitative Determination of Nanogram Amounts of Total and Oxidised Glutathione: Applications to Mammalian Blood and other Tissues.
Anal. Biochem. 27, 502-522, 1969.

(17) Higley B: Unpublished results.

(18) Crone C. The permeability of Capillaries in various Organs as Determined by use of the 'Indicator Dilution Method'. Acta physiol. scand. 58, 292-305, 1963.

(19) Winton F R, Bayliss L E. Human Physiology, 5th Edition, Churchill Press, 1962.

(20) Grassetti D R, Murray J F. Determination of sulhydryl groups with 2,2′ or 4,4′-dithiopyridine. Arch. Biochem. Biophys., 119, 41-49, 1967.

(21) Davson H, Eggleton G: Principles of Human Physiology, 1968 p 252

(22) Lassen N A, Andersen A R, et al: Technetium-99m HM-PAO as a Tracer of Cerebral Blood Flow Distribution: A Kinetic Analysis. Submitted to J. Nucl. Med.

(23) Crone C, Thompson A M. Permeability of Brain Capilliaries in Capillary Permeability. Crone C and Lassen N A (Eds.). Scandinavian University Books, Copenhagen, 1970, pp 447-453.

(24) Ketty SS. The Theory and Applications of the Exchange of Inert Gas at the Lungs and Tissues. Pharmacol. Rev., 3, 1-41, 1951.

(25) Snyder W S et al. Report of the Task Group on Reference Man. ICRP Publication 23, Permagon Press, Oxford, 1975.

(26) Fernandez V, Videla L A. Effect of Acute and Chronic Ethanol Ingestion on the Content of Reduced Glutathione of Various Tissues of the Rat. Experientia, 37, 392-394 (1981).

(27) Ecobichon D J. Glutathione Depletion and Resynthesis in Laboratory Animals. Drug Chem.Toxicol.(US), 345-355 (1984).

(28) Kaplan E, DeMaster E G, Nagasawa H T. Effect of Pargylene on Hepatic Glutathione Levels in Rats treated acutely and Chronically with Ethanol
Res. Commun. Chem. Pathol. Pharmacol, 30(3), 577-580, (1980)

(29) Guerri C, Grisolia S. Changes in Glutathione in Acute and Chronic Alcohol Intoxication. Pharmacol.Biochem.Behav. 13 Suppl.1, 53-61 (1980).

(30) Tom W M, Prasongwatana V, Boyde T R C. The effects of Vitamin A Nutritional Status on Glutathione Levels and Microsomal Lipid Peroxidation in Rat Lung. Experientia, 41(8), 1046-1047,(1985).

(31) Thomson C G, Martin H. Techniques for Determining Glutathione in Animal Tissues. In Glutathione, Crook E M (Ed). Cambridge University Press, 1959, 17-25.

(32) McIlwain H. Glutathione and Neural Tissues. In Glutathione, Crook E M (Ed), Cambridge University Press, 1959, 66-78.

(33) Meister A. Biochemistry of glutathione. In Metabolism of Sulphur Compounds, Greenberg D M (Ed), Academic Press, 1975, 101-188.

(34) Chasseaud L F. Conjugation with glutathione and mercapturic acid excretion. In Glutathione: Metabolism and Function, Arias I M and Jakoby W M (Eds), Raven Press, New York, 1976, 77-114.

(35) Meister A Metabolism and Transport of Glutathione and other γ-Glutamyl Compounds. In Functions of Glutathione, Biochemical, Physiological, Toxicological and Clinical Aspects. Larsson A, Holmgren A, Orrenius S and Mannervick B (Eds), Raven Press, New York, 1983, 1-22.

(36) Meister A. Glutathione: Metabolism and Function via the γ-glutamyl cycle. Life Sci. 15(2), 177-190.

**TABLE 1 : VALUES OF f, E, ρ, $k_2$, T and [GSH]**

| Organ | f ($mlg^{-1}min^{-1}$) | E | ρ ($gml^{-1}$) | $k_2$ ($min^{-1}$) | T (min) | [GSH] (mM) |
|---|---|---|---|---|---|---|
| Brain | 0.54 | 0.90 | 1.03 | 0.50 | 0.3 | 2.3 |
| Lung | 11.7 | 1.0+ | 1.05 | 12.3 | 0.17 | 1.6 |
| Heart | 0.70 | 1.0 | 1.03 | 0.72 | 0.2 | 1.9 |
| Liver | 0.54 | 1.0 | 1.04 | 0.56 | 0.3 | 7.0 |
| G I tract | 1.80 | 1.0 | 1.04 | 1.9 | * | 2.5 |

+ See text

* Value not required

**TABLE 2 : VALUES OF $k_3$, $k_5$ AND R**

| Complex | Organ | $k_{GSH}$* | $k_3$ ($min^{-1}$) | $k_5$ ($min^{-1}$) | R |
|---|---|---|---|---|---|
| Tc-99m d,1 HM-PAO | Brain | H | 0.78 | | 0.61 |
| | | M | 0.48 | | 0.49 |
| | | L | 0.28 | | 0.36 |
| | Lung | H | 0.55 | | 0.044 |
| | | M | 0.33 | | 0.026 |
| | | L | 0.20 | | 0.016 |
| | Heart | H | 0.65 | | 0.47 |
| | | M | 0.40 | | 0.35 |
| | | L | 0.23 | | 0.24 |
| | Liver | H | 2.39 | | 0.81 |
| | | M | 1.46 | | 0.72 |
| | | L | 0.85 | | 0.60 |
| | GI Tract | H | 0.85 | | 0.31 |
| | | M | 0.52 | | 0.22 |
| | | L | 0.31 | | 0.14 |
| | Blood | H | | 0.41 | |
| | | M | | 0.25 | |
| | | L | | 0.15 | |
| Tc-99m meso HM-PAO | Brain | H | 0.069 | | 0.12 |
| | | M | 0.042 | | 0.077 |
| | | L | 0.025 | | 0.047 |
| | Lung | H | 0.048 | | 0.0039 |
| | | M | 0.029 | | 0.0024 |
| | | L | 0.017 | | 0.0014 |
| | Blood | H | | 0.036 | |
| | | M | | 0.022 | |
| | | L | | 0.013 | |

* H: $k_{GSH}$ = 341 (d,1) or 29.9 (meso) $l\ mol^{-1}\ min^{-1}$
M: $k_{GSH}$ = 208 (d,1) or 18.2 (meso) $l\ mol^{-1}\ min^{-1}$
L: $k_{GSH}$ = 122 (d,1) or 10.7 (meso) $l\ mol^{-1}\ min^{-1}$

**TABLE 3 : CALCULATION OF THE EXPECTED HUMAN ORGAN DISTRIBUTION AND COMPARISON WITH CLINICAL RESULTS**

| Complex | L (%) | Organ | C (%) | E | $k_{GSH}$* | V | $e^{-k_5T}$ | R | Percentage of inj. material in organ: Calculated | Percentage of inj. material in organ: Found |
|---|---|---|---|---|---|---|---|---|---|---|
| Tc99m d,1 HM-PAO | 87 | Brain | 12 | 0.90 | H | 0.96 | 0.89 | 0.61 | 4.9 ) | 4.9±1.3 |
| | | | | | M | 0.97 | 0.93 | 0.49 | 4.1 ) | |
| | | | | | L | 0.98 | 0.96 | 0.36 | 3.2 ) | |
| | | Lung | 100 | 1.0+ | H | 1.00 | 0.93 | 0.044 | 3.5 ) | 7.4±4.9 |
| | | | | | M | 1.00 | 0.96 | 0.026 | 2.2 ) | |
| | | | | | L | 1.00 | 0.98 | 0.016 | 1.3 ) | |
| | | Heart | 3 | 1.0 | H | 0.96 | 0.92 | 0.47 | 1.1 ) | 1-2 |
| | | | | | M | 0.97 | 0.95 | 0.35 | 0.9 ) | |
| | | | | | L | 0.98 | 0.97 | 0.24 | 0.6 ) | |
| | | Liver | 30 | 1.0 | H | 0.73 | 0.89 | 0.81 | 13.7 ) | 12.0±4.4 |
| | | | | | M | 0.82 | 0.93 | 0.72 | 14.3 ) | |
| | | | | | L | 0.88 | 0.96 | 0.60 | 13.3 ) | |
| Tc-99m meso HM-PAO | 92 | Brain | 12 | 0.90 | H | 1.00 | 0.99 | 0.12 | 1.2 ) | 1.6,1.8 |
| | | | | | M | 1.00 | 1.00 | 0.077 | 0.8 ) | |
| | | | | | L | 1.00 | 1.00 | 0.047 | 0.5 ) | |
| | | Lung | 100 | 1.0+ | H | 1.00 | 0.99 | 0.0039 | 0.4 ) | Not detect-able |
| | | | | | M | 1.00 | 1.00 | 0.0024 | 0.2 ) | |
| | | | | | L | 1.00 | 1.00 | 0.0014 | 0.1 ) | |

* H: $k_{GSH}$ = 341 (d,1) or 29.9 (meso) $1\ mol^{-1}\ min^{-1}$
M: $k_{GSH}$ = 208 (d,1) or 18.2 (meso) $1\ mol^{-1}\ min^{-1}$
L: $k_{GSH}$ = 122 (d,1) or 10.7 (meso) $1\ mol^{-1}\ min^{-1}$

\+ See text

## Claims

1. A method of preparing a formulation for the detection or treatment of regions of relatively high (or low) glutathione concentration, which method comprises bringing into solution a neutral lipophilic complex of a propylene amine oxime ligand having the property of undergoing <u>in vivo</u> a chemical reaction mediated by glutathione resulting in loss of the ability to pass through cell membranes.

2. A method involving the use of a neutral lipophilic complex of a propylene amine oxime ligand having the property of undergoing in vivo a chemical reaction midiated by glutathione resulting in loss of the ability to pass through the cell membranes, which method comprises administering to a patient the neutral lipophilic complex which undergoes the chemical reaction mediated by glutathione and as a result becomes potentially located in vivo in regions of relatively high (or low) glutathione concentration.

3. A method as claimed in claim 1 or claim 2, wherein the complex is a Tc-99m complex.

4. A method as claimed in any one of claims 1 to 3, wherein the propylene amine oxime has the formula 3:-

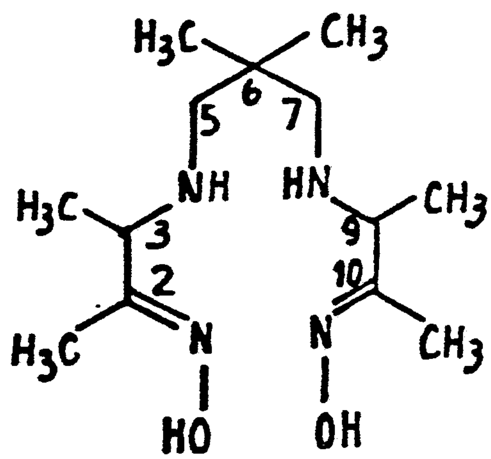

3

5. A method as claimed in claim 4, wherein the propylene amine oxime of formula 3 is used in its d,l-form.

6. A method as claimed in claim 4, wherein the propylene amine oxime of formula 3 is used in its meso-form.

7. A method as claimed in any one of claims 2 to 6 wherein the complex is a Tc-99m complex, comprising the additional step of observing the location of the Tc-99m in vivo.

8. A method as claimed in claim 7, comprising the additional step of deducing glutathione concentrations at locations in vivo.

9. A method as claimed in claim 8, comprising the additional step of using the glutathione concentrations to detect tumours or other damage or disease at the locations.

10. Use of a neutral lipophilic complex of a propylene amine oxime ligand of formula 3 in its meso-form, for the manufacture of a composition for detecting or treating tumours or other damaged or diseased tissues.

European Patent Office

# EUROPEAN SEARCH REPORT

Application Number

EP 88 30 0652

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | JOURNAL OF NUCLEAR MEDICINE, vol. 24, no. 5, May 1983, page 10, New York, US; D.E. TROUTNER et al.: " A tetradentate amine oxime complex of TC-99m" * whole article * --- | 1-10 | A 61 K 49/02 |
| Y,D | EP-A-0 194 843 (AMERSHAM) * claims, page 30-31 * --- | 1-10 | |
| Y,D | EP-A-0 123 504 (THE CURATORS OF THE UNIVERSITY OF MISSOURI) * claims * --- | 1-10 | |
| Y,D | THE LANCET, no. 8445, July 1985, pages 50-51, London, GB; P.J. ELL et al.: " Regular cerebral blood flow mapping with 99mTc-labelled compound" * whole article * --- | 1-10 | |
| Y | THE LANCET, no. 8513, October 1986, pages 946-949, London, GB; A.M. PETERS et al.: "Clinical experience with 99mTc-Hexamethylpropyleneamine oxime for labelling leucocytes and imaging inflammation" * results * --- | 1-10 | **TECHNICAL FIELDS SEARCHED (Int. Cl.4)** A 61 K 49/00 |
| Y,D | THE LANCET, no. 8469/70, December 1985, pages 1436-1437, London, GB; H. BIERSACK et al.: "99mTc-labelled Hexamethylpropyleneamine oxime photon emission scans in epilepsy" * whole article * ----- | 1-10 | |

The present search report has been drawn up for all claims

| Place of search | Date of completion of the search | Examiner |
|---|---|---|
| BERLIN | 20-05-1988 | AVEDIKIAN P.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)